# EUROPÄISCHE PATENTANMELDUNG

(11) **EP 2 591 803 A1**
(43) Veröffentlichungstag der Anmeldung: **15.05.2013**
(21) Anmeldenummer: 11188495.3
(22) Anmeldetag: 09.11.2011
(51) Int. Cl.: A61K 41/00, A61K 47/42, A61P 35/00

(54) **Zusammensetzung für die photodynamische Diagnostik und Therapie von Tumoren**

(71) Anmelder: APOCARE Pharma GmbH, 33647 Bielefeld (DE)
(72) Erfinder: Die Erfindernennung liegt noch nicht vor
(74) Vertreter: Dey, Michael

(57) **Zusammenfassung**

Die vorliegende Erfindung betrifft eine Zusammensetzung, welche insbesondere in der photodynamischen Diagnostik oder/und Therapie von Tumoren eingesetzt werden kann. Weiterhin betrifft die Erfindung die Verwendung von Albuminen zur Stabilisierung von Chlorin e6 oder pharmazeutisch annehmbaren Derivaten hiervon.

## Beschreibung

Die vorliegende Erfindung betrifft eine Zusammensetzung, welche insbesondere in der photodynamischen Diagnostik oder/und Therapie von Tumoren eingesetzt werden kann. Weiterhin betrifft die Erfindung die Verwendung von Albuminen zur Stabilisierung von Chlorin e6 oder pharmazeutisch annehmbaren Derivaten hiervon.

Die photodynamische Diagnostik (PDD) und die photodynamische Therapie (PDT) stellen Verfahren zur Erkennung bzw. Behandlung von Tumoren und anderen Gewebeveränderungen dar, welche auf einer Bestrahlung des betroffenen Gewebes mit Licht geeigneter Wellenlänge basieren. Hierbei wird dem Patienten vor der Bestrahlung ein primär nicht-toxischer Photosensibilisator verabreicht, welcher sich aufgrund einer Reihe von Faktoren (spezifische Transporter, erhöhter Tumorzellstoffwechsel, verstärkte Blutgefäßversorgung, geringer lymphatischer Abfluß) spezifisch in Tumorzellen anreichert.

Nach der maximalen Anreicherung des Photosensibilisators im Tumorgewebe kann dieser durch Bestrahlung mit Licht geeigneter Wellenlänge angeregt werden. Im angeregten Zustand überträgt der Photosensibilisator Energie auf einen geeigneten Reaktionspartner, wie beispielsweise auf im Gewebe vorhandenen molekularen Sauerstoff. Der hierbei entstehende Singulett-Sauerstoff stellt ein starkes Oxidationsmittel dar, welches zelluläre Strukturen von Tumorzellen durch Oxidation so stark schädigt, dass dies zur Apoptose oder Nekrose führt.

Photosensibilisatoren, welche für eine Anwendung im Bereich der photodynamischen Diagnostik bzw. Therapie in Frage kommen, umfassen Hämatoporphyrine, Phthalocyanine und Naphthalocyanine. Aufgrund geringer Toxizität und einer effizienten Anregbarkeit im sichtbaren Spektralbereich haben sich in der Praxis insbesondere Photosensibilisatoren auf Porphyrinbasis bewährt, welche u.a. Chlorin e6 und Derivate hiervon umfassen.

Chlorin e6 besitzt mehrere intensive Absorptionsbanden. Von praktischer Bedeutung sind hierbei insbesondere die sogenannte Soretbande im Spektralbereich von 400 ± 10 nm, welche für die photodynamische Diagnostik Relevanz besitzt, sowie eine Absorptionsbande bei 660 ± 10nm, welche aufgrund einer höheren Eindringtiefe in das Gewebe für die photodynamische Therapie genutzt wird. Gleichzeitig besitzt Chlorin e6 den Vorteil, dass es im Körper vergleichsweise schnell metabolisiert und ausgeschieden wird; 48 Stunden nach der Verabreichung sind nur noch Spuren der verabreichten Wirksubstanz im menschlichen Organismus nachzuweisen.

Allerdings ergibt sich für Chlorin e6 das Problem, dass die Substanz in wässriger Lösung instabil ist. Infolge dessen kann eine gebrauchsfertige wässrige Lösung von Chlorin e6 nicht über längere Zeiträume unter üblichen Bedingungen, d.h. im Kühlschrank oder bei Raumtemperatur, gelagert werden, ohne dass ein signifikanter Abbau der Substanz erfolgt, der zu einem geringerem Wirkstoffgehalt und zur Entstehung unerwünschter Zersetzungsprodukte führt.

Die der vorliegenden Erfindung zugrundeliegende Aufgabe bestand somit darin, eine Zusammensetzung enthaltend Chlorin e6 oder ein pharmazeutisch annehmbares Derivat hiervon bereitzustellen, bei welcher die Nachteile des Standes der Technik zumindest teilweise beseitigt sind. Insbesondere sollte die Zusammensetzung über einen längeren Zeitraum, z.B. über einen Zeitraum von mehreren Wochen bei Raumtemperatur bzw. über einen Zeitraum von mehreren Monaten bei Kühlschranktemperatur, gelagert werden können, ohne dass der Wirkstoff in signifikantem Ausmaß zersetzt wird.

Diese Aufgabe wurde erfindungsgemäß gelöst durch eine Zusammensetzung, welche neben Chlorin e6 oder einem pharmazeutisch annehmbaren Derivat hiervon obligatorisch ein Albumin umfasst.

Überraschenderweise wurde gefunden, dass mit Hilfe von Albuminen eine Langzeitstabilisierung von Chlorin e6 oder pharmazeutisch annehmbaren Derivaten hiervon möglich ist. Bei dem Albumin kann es sich grundsätzlich um ein beliebiges Albumin natürlichen oder synthetischen Ursprungs handeln, wobei Humanalbumine bevorzugt sind. Bevorzugt handelt es sich bei dem Albumin um humanes Serumalbumin (HSA).

Der Ausdruck "pharmazeutisch annehmbares Derivat", wie er in vorliegender Anmeldung verwendet wird, bezeichnet einen Abkömmling eines Wirkstoffs, welcher für den Empfänger im wesentlichen unschädlich ist und die biologische Wirksamkeit bzw. die biologischen Eigenschaften des Wirkstoffs besitzt. Beispiele für pharmazeutisch annehmbare Derivate im Sinne der vorliegenden Anmeldung umfassen pharmazeutisch annehmbare Salze, Ester und Amide. In einer bevorzugten Ausführungsform handelt es sich bei dem pharmazeutisch annehmbaren Derivat um ein pharmazeutisch annehmbares Salz, wie beispielsweise das Trinatriumsalz von Chlorin e6.

Der Begriff "Langzeitstabilisierung", wie er in vorliegender Anmeldung verwendet wird, bedeutet, dass Chlorin e6 für eine beliebige Zeitspanne, bevorzugt über einen Zeitraum von mindestens 4 Wochen, stärker bevorzugt über einen Zeitraum von mindestens 6 Monaten, und am stärksten bevorzugt über einen Zeitraum von mindestens 12 Monaten in Gegenwart eines Albumins gelagert wird, wobei das Gemisch aus Chlorin e6 und Albumin nach der Lagerung mehr als 65 Gew.-%, bevorzugt mehr als 80 Gew.-%, besonders bevorzugt mehr als 90 Gew.-% der Ausgangsmenge an Chlorin e6 enthält. Die Lagerung erfolgt vorzugsweise bei Atmosphärendruck, bei einer relativen Luftfeuchtigkeit von mindestens 50%, und bei einer Temperatur im Bereich von etwa 2°C bis etwa 25°C (Raumtemperatur), wobei sich eine Lagerung bei 2-8°C als besonders vorteilhaft erwiesen hat.

Die erfindungsgemäße Zusammensetzung kann Chlorin e6 und das Albumin grundsätzlich in jeder dem Fachmann geeignet erscheinenden Menge enthalten. Für die Zwecke der Erfindung ist es indessen bevorzugt, dass die Zusammensetzung Chlorin e6 in einer Menge von etwa 0.02 Gew.-% bis etwa 0.2 Gew.-%, stärker bevorzugt in einer Menge von etwa 0.04 Gew.-% bis etwa 0.16 Gew.-%, und am stärksten bevorzugt in einer Menge von etwa 0.06 Gew.-% bis etwa 0.12 Gew.-%, bezogen auf das Gesamtgewicht der Zusammensetzung, umfasst.

Demgegenüber umfasst die Zusammensetzung das Albumin bevorzugt in einer Menge von etwa 0.1 Gew.-% bis etwa 99 Gew.-%, stärker bevorzugt in einer Menge von etwa 1.0 Gew.-% bis etwa 20 Gew.-%, und am stärksten bevorzugt in einer Menge von etwa 2.5 Gew.-% bis etwa 15 Gew.-%, bezogen auf das Gesamtgewicht der Zusammensetzung.

Die erfindungsgemäße Zusammensetzung kann grundsätzlich beliebig formuliert sein, sofern die Formulierung dem Fachmann für den beabsichtigten Verwendungszweck geeignet erscheint. Beispiele für Formulierungen im Sinne der vorliegenden Anmeldung umfassen u.a. Aerosole, Lösungen, Schäume, Emulsionen, Suspensionen, Gele, Cremes, Salben, Lotionen, Tabletten und Zäpfchen, sind jedoch nicht auf diese beschränkt. Bevorzugt ist die Zusammensetzung als Lösung formuliert, wobei wässrige Lösungen als besonders bevorzugt gelten.

Die erfindungsgemäße Zusammensetzung kann, sofern sie in flüssiger Form vorliegt, grundsätzlich jeden vom Fachmann für sinnvoll erachteten pH-Wert aufweisen. Da die Stabilität von Chlorin e6 mit sinkendem pH-Wert abnimmt, ist es erfindungsgemäß allerdings bevorzugt, dass die Zusammensetzung einen pH-Wert von ≥ 6.0 aufweist. Stärker bevorzugt weist die Zusammensetzung einen pH-Wert im Bereich von 6.0 bis 10.0, noch stärker bevorzugt einen pH-Wert im Bereich von 7.0 bis 9.0 auf. Als besonders bevorzugt gilt ein pH-Wert im Bereich von 8.0 bis 9.0.

Neben Chlorin e6 und einem Albumin kann die Zusammensetzung, sofern gewünscht, weitere Komponenten enthalten, wie beispielsweise einen pharmazeutisch annehmbaren Träger oder/und Additive. In einer besonders bevorzugten Ausführungsform der Erfindung umfasst die Zusammensetzung keinerlei Pyrrolidon-Derivate, wie beispielsweise Polyvinylpyrrolidon.

Der Ausdruck "pharmazeutisch annehmbarer Träger", wie er in vorliegender Anmeldung verwendet wird, bezeichnet jegliche organische oder anorganische, natürliche oder synthetische Substanz, welcher zur Vereinfachung der Applikation eines Wirkstoffs mit diesem kombiniert werden kann und für eine Verabreichung an Säuger, einschließlich Menschen, geeignet ist. Beispiele für pharmazeutisch annehmbare Träger umfassen, sind jedoch nicht beschränkt auf, organische oder anorganische Lösungsmittel, Stärke, Laktose, Mannitol, Methylcellulose, Talk, Gelatine, Agar-Agar, Calciumphosphat, Magnesiumstearat, tierische und pflanzliche Fette, höhermolekulare Fettsäuren, oder höhermolekulare Polymere.

Additive im Sinne der vorliegenden Anmeldung umfassen beispielsweise pH-Puffer, Verdünnungsmittel, Verarbeitungshilfsmittel wie etwa Emulgatoren, Konservierungsmittel, Stabilisatoren, Antioxidationsmittel, Lichtschutzmittel, und Farbstoffe, sind jedoch nicht auf diese beschränkt. In einer bevorzugten Ausführungsform der Erfindung umfasst die Zusammensetzung neben Chlorin e6 und einem Albumin weiterhin einen pH-Puffer, welcher vom Fachmann entsprechend den Anforderungen an die zu applizierende Zusammensetzung ausgewählt werden kann. Beispielhafte pH-Puffer umfassen, sind jedoch nicht beschränkt auf, Bicarbonatpuffer, Phosphatpuffer, und dergleichen.

In einem weiteren Aspekt betrifft die Erfindung die Verwendung der erfindungsgemäßen Zusammensetzung in einem medizinischen Verfahren. Bevorzugt gelangt die erfindungsgemäße Zusammensetzung hierbei in der photodynamischen Diagnostik oder/und Therapie von dysplastischen Veränderungen, und besonders bevorzugt in der photodynamischen Diagnostik oder/und Therapie von Tumoren, zum Einsatz. Alternativ kann die erfindungsgemäße Zusammensetzung auch zur Bekämpfung von Bakterien eingesetzt werden, wobei der Begriff "Bekämpfung" sowohl eine bloße Inaktivierung als auch eine vollständige Abtötung umfasst.

Sofern die Zusammensetzung in der photodynamischen Diagnostik oder/und Therapie von Tumoren verwendet wird, so handelt es sich bei dem Tumor bevorzugt um einen Tumor der Blase, der Prostata, der Lunge, der Schleimhäute, des Hals-Kopf-Bereichs, oder der Haut. Besonders bevorzugt können mittels der Zusammensetzung Tumore der Haut, welche speziell Basaliome und Melanome umfassen, diagnostiziert oder/und behandelt werden.

In noch einem weiteren Aspekt betrifft die Erfindung die Verwendung von Albuminen zur Stabilisierung von Chlorin e6 oder pharmazeutisch annehmbaren Derivaten hiervon. Was bevorzugte Ausgestaltungen des Albumins betrifft, so wird auf die Ausführungen im Rahmen der Beschreibung der erfindungsgemäßen Zusammensetzung verwiesen.

Die Erfindung soll durch die nachfolgenden Figuren und Beispiele näher erläutert werden:

### Beschreibung der Figuren

**Figur 1****:** Stabilität verschiedener Zusammensetzungen gemäß der vorliegenden Erfindung bei einem pH-Wert von 7.0, 7.5 bzw. 8.0 nach Lagerung für 4 Tage bei 30°C.
**Figur 2****:** Stabilität verschiedener Zusammensetzungen gemäß der vorliegenden Erfindung bei einem pH-Wert von 7.0, 7.5 bzw. 8.0 nach Lagerung für 4 Wochen bei 30°C.
**Figur 3****:** Stabilität verschiedener Zusammensetzungen gemäß der vorliegenden Erfindung bei einem pH-Wert von 7.0, 7.5 bzw. 8.0 nach Lagerung für 10 Wochen bei 30°C.
**Figur 4****:** Stabilität verschiedener Zusammensetzungen gemäß der vorliegenden Erfindung bei einem pH-Wert von 8.0 nach Lagerung für 6 Monate bei 5°C.
**Figur 5****:** Stabilität verschiedener Zusammensetzungen gemäß der vorliegenden Erfindung bei einem pH-Wert von 8.0 nach Lagerung für 9 Monate bei 5°C.
**Figur 6****:** Stabilität verschiedener Zusammensetzungen gemäß der vorliegenden Erfindung bei einem pH-Wert von 8.0 nach Lagerung für 12 Monate bei 5°C.

### Beispiel

Zur Bestimmung der Stabilität einer Zusammensetzung, umfassend Chlorin e6 und ein Albumin, wurden zunächst drei Pufferlösungen mit pH-Werten von 7.0, 7.5 bzw. 8.0, welche jeweils Kaliumdihydrogenphosphat (Firma Merck) und Dinatriumhydrogenphosphat (Firma Merck) als Puffersalze enthielten, hergestellt und sterilisiert.

Ausgehend von einer 20 Gew.-% Stammlösung von humanem Serumalbumin (Firma Biotest AG) wurden mittels obiger Pufferlösungen anschließend jeweils verdünnte Lösungen mit 10 Gew.-%, 5 Gew.-% bzw. 0 Gew.-% an humanem Serumalbumin hergestellt. Zum Puffern der 20 Gew.-% Stammlösung von humanem Serumalbumin wurden die Puffersalze als Feststoffe direkt zu 20 ml Aliquoten der Stammlösung hinzugefügt.

In der Folge wurden 2 ml der verschiedenen gepufferten Lösungen jeweils in Braunglasflaschen mit Bördelverschluss eingebracht und mit 40 µl einer 75 mM Lösung von Chlorin e6-Natriumsalz (Fa. ORPEGEN Peptide Chemicals GmbH) in Wasser versetzt. Auf diese Weise wurden wässrige Lösungen von Chlorin e6-Natriumsalz mit einer Konzentration von 0 Gew.-%, 1.25 Gew.-%, 2.5 Gew.-%, 5 Gew.-%, 10 Gew.-% bzw. 20 Gew.-% an humanem Serumalbumin bereitgestellt, welche pH-Werte von 7.0, 7.5 bzw. 8.0 aufwiesen. Zwecks Reproduzierbarkeit der Ergebnisse wurden die einzelnen Lösungen jeweils mehrfach angesetzt.

Die Lösungen wurden lichtdicht umverpackt und bei 30°C in einem Trockenschrank bzw. bei 5°C in einem Kühlschrank inkubiert. Nach 4 Tagen, 4 Wochen und 10 Wochen Lagerung im Trockenschrank bzw. nach 6 Monaten Lagerung im Kühlschrank wurde jeweils eine Probe der jeweiligen Lösung genommen und mittels analytischer HPLC (Säule: C18, 250 mm x 4.5 mm (Dr. Maisch GmbH); Laufmittel: 80 mM Triethylammoniumphosphat-Puffer pH 2.25/Acetonitril) vermessen. Das jeweilige Ausmaß der Zersetzung von Chlorin e6 wurde durch einen Vergleich der Flächen unter dem zu Chlorin e6 gehörigen Peak im HPLC-Diagramm vor und nach der Lagerung berechnet.

Bei der Auswertung von Proben, welche nach 4 Tagen Lagerung genommen wurden, zeigte sich in einer auf pH 8 eingestellten reinen Pufferlösung, d.h. in Abwesenheit von humanem Serumalbumin, ein signifikanter Abbau von Chlorin e6 (59.1% Fläche). Da kommerziell erhältliche Lösungen von humanem Serumalbumin obligatorisch Na-caprylat und N-Acetyl-DL-Tryptophan als Additive enthalten, wurde ferner ein Versuch durchgeführt, in welchem der Einfluss der beiden Substanzen auf die Stabilität einer wässrigen Lösung von Chlorin e6 untersucht wurde. Dabei zeigte sich, dass durch Zugabe von Na-caprylat bzw. N-Acetyl-DL-Tryptophan der Wirkstoff gegenüber der reinen Pufferlösung eine leichte Stabilisierung erfährt (68.8% Fläche).

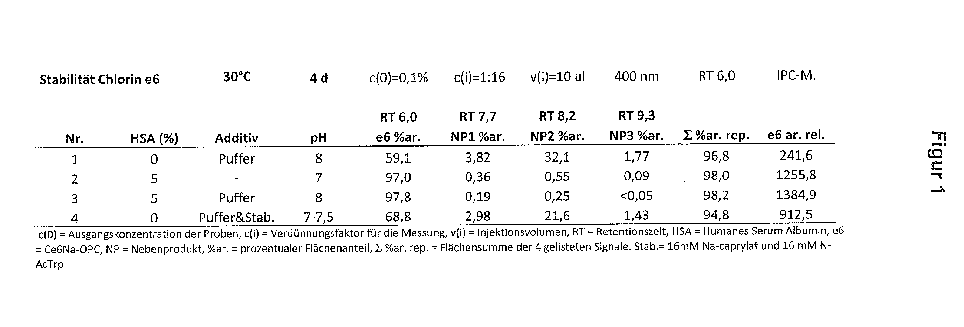

Der geringste Abbau von Chlorin e6 wurde in einer auf pH 8 eingestellten Pufferlösung beobachtet, welche 5 Gew.-% humanes Serumalbumin enthielt (97.8% Fläche). Im Falle einer Lagerung des Wirkstoffs in einer auf pH 7 eingestellten, nicht-gepufferten Lösung wurde in Gegenwart von 5% humanem Serumalbumin eine gegenüber Na-caprylat und N-Acetyl-DL-Tryptophan deutlich bessere Stabilisierung von Chlorin e6 beobachtet (97.0% Fläche). Die Ergebnisse sind in Figur 1 dargestellt.

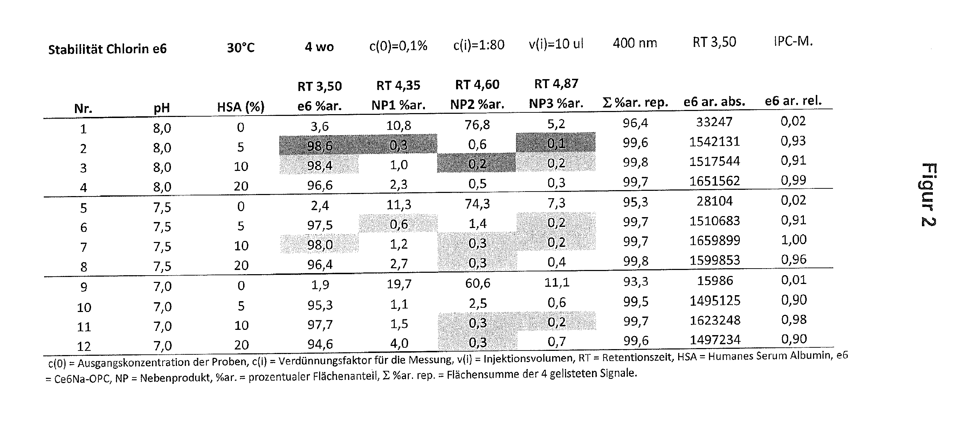

Die Vermessung von Proben nach 4 Wochen Lagerung im Trockenschrank zeigte im Falle reiner Pufferlösungen, d.h. ohne Zusatz von humanem Serumalbumin, einen nahezu vollständigen Abbau von Chlorin e6 (pH 7.0: 1.9% Fläche; pH 7.5: 2.4% Fläche; pH 8.0: 3.6% Fläche). Durch Zugabe von 5 Gew.-% an humanem Serumalbumin konnte die Zersetzung von Chlorin e6 bei allen drei pH-Werten signifikant reduziert werden, wie durch einen Restwirkstoffgehalt von > 95% belegt ist (pH 7.0: 95.3% Fläche; pH 7.5: 97.5% Fläche; pH 8.0: 98.6% Fläche). Bei Erhöhung der Menge an humanem Serumalbumin auf 10 Gew.-% wurde bei pH 7 und pH 7.5 ein etwas höherer Restwirkstoffgehalt beobachtet; bei Inkubation mit 20 Gew.-% humanem Serumalbumin sank der Restgehalt an Chlorin e6 bei allen drei pH-Werten leicht (pH 7.0: 94.6% Fläche; pH 7.5: 96.4% Fläche; pH 8.0: 96.6% Fläche). Die Ergebnisse sind in Figur 2 dargestellt.

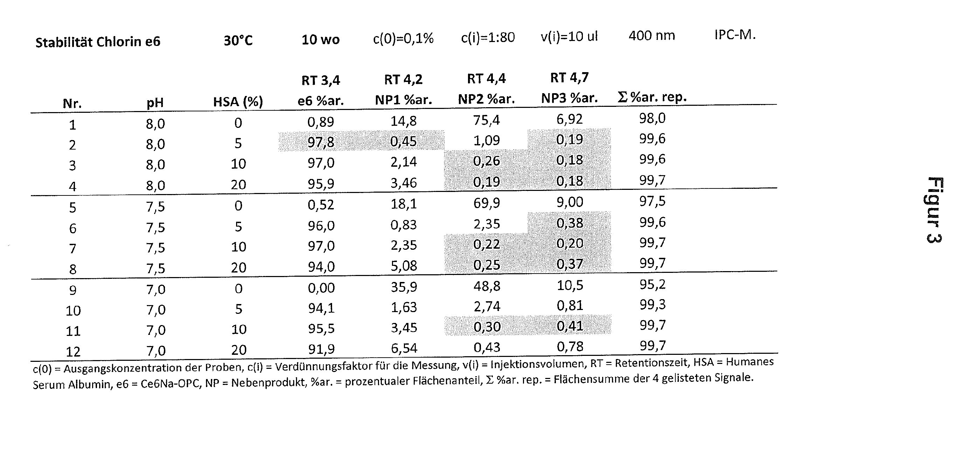

Die Vermessung von Proben nach 10 Wochen Lagerung bei 30°C zeigte im Falle reiner Pufferlösungen einen quasi vollständigen Abbau von Chlorin e6 (pH 7.0: 0% Fläche; pH 7.5: 0.52% Fläche; pH 8.0: 0.89% Fläche). Durch Zugabe von 5 Gew.-% an humanem Serumalbumin konnte die Zersetzung von Chlorin e6 bei allen drei pH-Werten wiederum signifikant reduziert werden, wie durch einen Restwirkstoffgehalt von > 94% belegt ist (pH 7.0: 94.1% Fläche; pH 7.5: 96.0% Fläche; pH 8.0: 97.8% Fläche). Bei Erhöhung der Menge an humanem Serumalbumin auf 10 Gew.-% wurde bei pH 7 und pH 7.5 erneut ein etwas höherer Restwirkstoffgehalt beobachtet; bei Inkubation mit 20 Gew.-% humanem Serumalbumin sank der Restgehalt an Chlorin e6 bei allen drei pH-Werten (pH 7.0: 91.9% Fläche; pH 7.5: 94.0% Fläche; pH 8.0: 95.9% Fläche). Die Ergebnisse, welche die Ergebnisse nach 4 Wochen auf etwas niedrigerem Chlorin e6-Niveau widerspiegeln, sind in Figur 3 dargestellt.

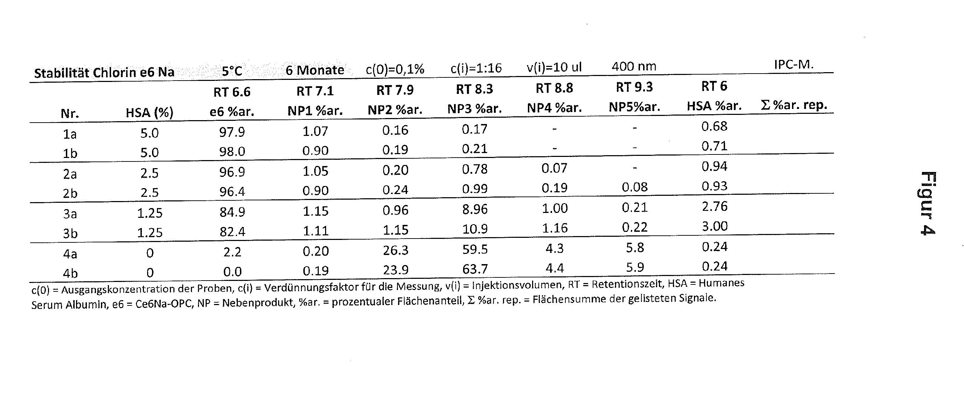

Die Vermessung von Proben nach 6 Monaten Lagerung bei 5°C und pH 8.0 zeigte im Falle reiner Pufferlösungen einen nahezu vollständigen Abbau von Chlorin e6 (1.1% Fläche im Mittel). Durch Zugabe von 5 Gew.-% an humanem Serumalbumin konnte die Zersetzung von Chlorin e6 signifikant reduziert werden, wie durch einen Restwirkstoffgehalt von > 97% belegt ist (97.9% Fläche im Mittel). Bei Erniedrigung der Menge an humanem Serumalbumin auf 2.5 Gew.-% wurde ein etwas niedrigerer Restwirkstoffgehalt von etwa 96.6% (Mittelwert) beobachtet; bei Inkubation mit 1.25 Gew.-% humanem Serumalbumin sank der Restgehalt an Chlorin e6 auf etwa 83.6% (Mittelwert). Die Ergebnisse sind in Figur 4 dargestellt.

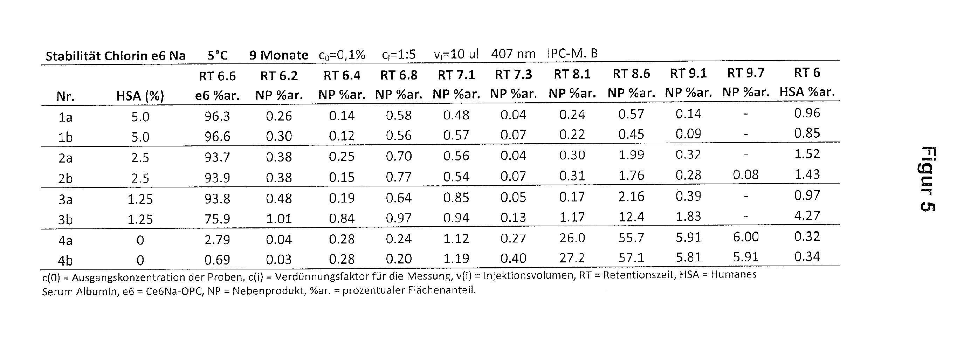

Die Vermessung von Proben nach 9 Monaten Lagerung bei 5°C und pH 8.0 zeigte im Falle reiner Pufferlösungen ebenfalls einen nahezu vollständigen Abbau von Chlorin e6 (1.74% Fläche im Mittel). Durch Zugabe von 5 Gew.-% an humanem Serumalbumin konnte die Zersetzung von Chlorin e6 signifikant reduziert werden, wie durch einen Restwirkstoffgehalt von > 96% belegt ist (96.4% Fläche im Mittel). Bei Erniedrigung der Menge an humanem Serumalbumin auf 2.5 Gew.-% wurde ein etwas niedrigerer Restwirkstoffgehalt von etwa 93.8% (Mittelwert) beobachtet; bei Inkubation mit 1.25 Gew.-% humanem Serumalbumin sank der Restgehalt an Chlorin e6 auf etwa 84.9% (Mittelwert). Die Ergebnisse sind in Figur 5 dargestellt.

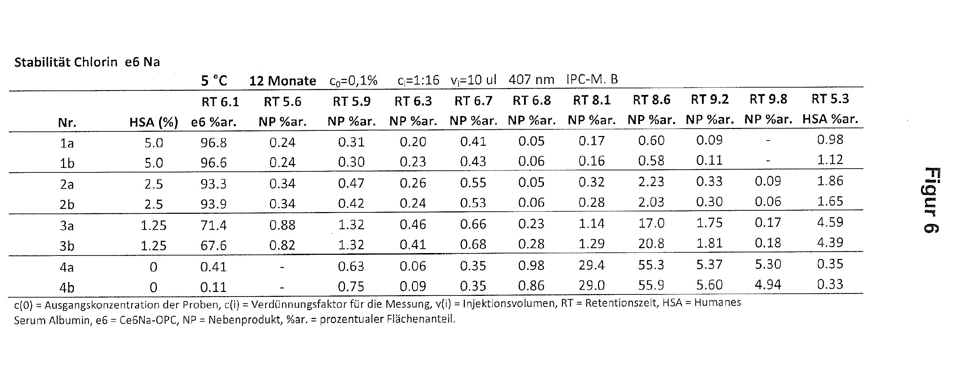

Die Vermessung von Proben nach 12 Monaten Lagerung bei 5°C und pH 8.0 zeigte im Falle reiner Pufferlösungen einen praktisch vollständigen Abbau von Chlorin e6 (0.26% Fläche im Mittel). Durch Zugabe von 5 Gew.-% an humanem Serumalbumin konnte die Zersetzung von Chlorin e6 signifikant reduziert werden, wie durch einen Restwirkstoffgehalt von > 96% belegt ist (96.7% Fläche im Mittel). Bei Erniedrigung der Menge an humanem Serumalbumin auf 2.5 Gew.-% wurde ein etwas niedrigerer Restwirkstoffgehalt von etwa 93.6% (Mittelwert) beobachtet; bei Inkubation mit 1.25 Gew.-% humanem Serumalbumin sank der Restgehalt an Chlorin e6 auf etwa 69.5% (Mittelwert). Die Ergebnisse sind in Figur 6 dargestellt.

Zusammenfassend bedeutet dies, dass bereits geringe Mengen an humanem Serumalbumin eine Chlorin e6-enthaltende wässrige Lösung drastisch stabilisieren. Sofern die Lagerung der Zusammensetzung bei einer Temperatur von 2-8°C anstelle von 30°C erfolgt, lässt sich aus den in den Figuren 1-6 gezeigten experimentellen Daten eine Haltbarkeit von mindestens 12 Monaten für eine wässrige Lösung von Chlorin e6 extrapolieren, womit die Zusammensetzung eine für medizinische Anwendungen geeignete Langzeitstabilität besitzt.

## Patentansprüche

1. Zusammensetzung, umfassend
(a) Chlorin e6 oder ein pharmazeutisch annehmbares Derivat hiervon, und
(b) ein Albumin.

2. Zusammensetzung nach Anspruch 1,
**dadurch gekennzeichnet,**
**dass** es sich bei dem Albumin um humanes Serumalbumin handelt.

3. Zusammensetzung nach Anspruch 1 oder 2,
**dadurch gekennzeichnet,**
**dass** sie Chlorin e6 in einer Menge von etwa 0.02 Gew.-% bis etwa 0.2 Gew.-%, bevorzugt in einer Menge von etwa 0.06 Gew.-% bis etwa 0.12 Gew.-%, bezogen auf das Gesamtgewicht der Zusammensetzung, umfasst.

4. Zusammensetzung nach einem der Ansprüche 1 bis 3,
**dadurch gekennzeichnet,**
**dass** sie das Albumin in einer Menge von etwa 0.1 Gew.-% bis etwa 99 Gew.-%, bevorzugt in einer Menge von etwa 2.5 Gew.-% bis etwa 15 Gew.-%, bezogen auf das Gesamtgewicht der Zusammensetzung, umfasst.

5. Zusammensetzung nach einem der Ansprüche 1 bis 4,
**dadurch gekennzeichnet,**
**dass** sie als wässrige Lösung formuliert ist.

6. Zusammensetzung nach einem der Ansprüche 1 bis 5,
**dadurch gekennzeichnet,**
**dass** sie einen pH-Wert im Bereich von 6.0 bis 10.0, bevorzugt im Bereich von 8.0 bis 9.0, aufweist.

7. Zusammensetzung nach einem der Ansprüche 1 bis 6,
**dadurch gekennzeichnet,**
**dass** sie weiterhin einen pH-Puffer umfasst.

8. Zusammensetzung nach einem der Ansprüche 1 bis 7,
**dadurch gekennzeichnet,**
**dass** sie kein Pyrrolidon-Derivat umfasst.

9. Zusammensetzung nach einem der Ansprüche 1 bis 8 zur Verwendung in einem medizinischen Verfahren.

10. Zusammensetzung nach einem der Ansprüche 1 bis 8 zur Verwendung in der photodynamischen Diagnostik oder/und Therapie von Tumoren.

11. Zusammensetzung nach Anspruch 10,
**dadurch gekennzeichnet,**
**dass** es sich bei dem Tumor um einen Tumor der Blase, der Prostata, der Lunge, der Schleimhäute, des Hals-Kopf-Bereichs oder der Haut, insbesondere der Haut, handelt.

12. Zusammensetzung nach einem der Ansprüche 1 bis 8 zur Verwendung in einem Verfahren zur Bekämpfung von Bakterien.

13. Verwendung eines Albumins zur Stabilisierung von Chlorin e6 oder einem pharmazeutisch annehmbaren Derivat hiervon.

14. Verwendung nach Anspruch 12,
**dadurch gekennzeichnet,**
**dass** es sich bei dem Albumin um humanes Serumalbumin handelt.
